# EUROPÄISCHE PATENTSCHRIFT

(11) **EP 3 755 397 B1**
(45) Veröffentlichungstag und Bekanntmachung des Hinweises auf die Patenterteilung: **02.11.2022**
(21) Anmeldenummer: 19706456.1
(22) Anmeldetag: 14.02.2019
(51) Int. Cl.: A61M 1/28, A61M 1/16, A61J 1/14

(54) **VORRICHTUNG ENTHALTEND DIALYSELÖSUNG ODER DIALYSELÖSUNGSKONZENTRAT**
DEVICE CONTAINING DIALYSATE OR DIALYSATE CONCENTRATE
DISPOSITIF CONTENANT DU DIALYSAT OU DU CONCENTRÉ POUR LE DIALYSAT

(30) Priorität: 21.02.2018 DE 102018103937
(43) Veröffentlichungstag der Anmeldung: 30.12.2020
(73) Patentinhaber: Fresenius Medical Care Deutschland GmbH, 61352 Bad Homburg (DE)
(72) Erfinder: WABEL, Peter, 61191 Rosbach (DE); BERLICH, Robert, 66606 St. Wendel (DE); BREUNINGER, Marcus, 61352 Bad Homburg (DE); STAUDE, Birgit, 64319 Pfungstadt (DE); RAU, Matthias, 65195 Wiesbaden (DE); WOLF, Klaus, 97450 Muedesheim (DE)
(74) Vertreter: Herrmann, Uwe
(86) Internationale Anmeldenummer: PCT/EP2019/053641
(87) Internationale Veröffentlichungsnummer: WO 2019/162178

(56) Entgegenhaltungen:
- DE-A1- 19 520 916
- US-A1- 2014 276 373

## Beschreibung

Die Erfindung betrifft eine Vorrichtung enthaltend eine Dialyselösung oder ein Dialyselösungskonzentrat, vorzugsweise zur Verwendung in der Peritonealdialyse, mit einem Aufnahmebehältnis, das ein Lösungsvolumen fassen kann, welches für wenigstens zwei Behandlungen bestimmt ist.

Insbesondere im Bereich der Peritonealdialyse ist es üblich, dem Patienten Lösungsbeutel zur Verfügung zu stellen, die mit einer für den Patienten geeigneten Dialyselösung befüllt sind. Diese Lösungsbeutel schließt der Patient dann selbsttätig oder mit Unterstützung von medizinischem Fachpersonal an einen Einlaufschlauch, d.h. an den Patientenkatheter an, um das Peritoneum mit der Lösung zu füllen.

Die Dialyselösung muss typischerweise in fertig zubereitetem Zustand zum Haus des Patienten transportiert werden. US2014/276373A1 offenbart ein Peritonealdialysegerät, das ein Aufnahmebehältnis aufweist, das ein für wenigstens zwei Behandlungen bestimmtes Dialyselösungsvolumen fassen kann, wobei das Gerät mehrere Entnahmeanschlüsse zur Entnahme der Dialyselösung aus dem Aufnahmebehältnis aufweist.

Es wäre wünschenswert, ein Lösungsvolumen für mehrere Behandlungen in einem Behältnis, beispielsweise Beutel unterzubringen, um so eine Abfallreduktion erreichen zu können und den Herstellungsprozess und die Qualitätskontrolle insofern vereinfachen zu können, dass weniger Behältnisse befüllt werden müssen. Auch der Transport und die Handhabung können für den Patienten unter Umständen einfacher sein, wenn lediglich ein Behältnis hantiert werden muss.

In der WO 2010/096657 A1 wird bereits der Gedanke offenbart, ein Behältnis mit einem Volumen an Peritonealdialyselösung zu befüllen, das für mehrere Behandlungen bestimmt ist. Dort wird ein Dosierbeutel zwischen das große Behältnis und den Patienten geschaltet, um die Dosierung der richtigen Flüssigkeitsmenge für eine Behandlung zu vereinfachen. Jedoch fehlt es bisher an geeigneten Maßnahmen, um die Sterilität des Entnahmeanschlusses sicherzustellen, wenn mehrmals Dialyselösung aus demselben Behältnis entnommen werden soll.

Aufgabe der Erfindung ist es, ein geeignetes Anschlusskonzept zur mehrmaligen Entnahme von Dialyselösung aus demselben Aufnahmebehältnis aufzufinden, bei dem insbesondere auch nach der ersten Entnahme und weiteren Entnahmen die Sterilität des Entnahmeanschlusses sicher gewährleistet ist.

Vor diesem Hintergrund betrifft die Erfindung eine Vorrichtung enthaltend eine Dialyselösung oder ein Dialyselösungskonzentrat, vorzugsweise zur Verwendung in der Peritonealdialyse, mit einem Aufnahmebehältnis, das ein Lösungsvolumen fassen kann, welches für wenigstens zwei Behandlungen bestimmt ist, wobei die Vorrichtung mehrere Entnahmeanschlüsse zur Entnahme der Dialyselösung aus dem Aufnahmebehältnis aufweist und die Entnahmeanschlüsse eine Doppelversicherung aufweisen, die den Entnahmeanschluss nach Verwendung irreversibel verschließt.

Unter einer Behandung wird im Zusammenhang mit der Peritonealdialyse eine vollständige oder teilweise Füllung des Bauchraums des Patienten verstanden. Unter einem Entnahmeanschluss wird im gegebenen Zusammenhang eine Schnittstelle zum direkten oder mittelbaren Anschließen der in den Bauchraum des Patienten führenden Leitung, d.h. dem Patientenkatheter verstanden. Der Entnahmeanschluss kann zu diesem Zweck beispielsweise mit einem Luer-Konnektor versehen sein.

Durch die Mehrzahl an Anschlüssen kann bei jeder von mehreren Entnahmen von Dialyselösung aus dem Aufnahmebehältnis ein frischer Entnahmeanschluss verwendet werden.

Das Aufnahmebehältnis kann ein Volumen von größer 5 Litern, größer 10 Litern oder auch größer 20 Litern aufweist. Das typischerweise im Rahmen einer Einzelbehandlung benötigte Volumen liegt in der Größenordnung von etwa 2 Litern. Die Anzahl der Anschlüsse kann auf die Größe des Aufnahmebehältnisses abgestimmt sein. Beispielsweise kann die Vorrichtung wenigstens einen Entnahmeanschluss pro zwei Liter Volumen des Aufnahmebehältnisses aufweisen.

Außer den Anschlüssen zur Entnahme der Dialyselösung weist das Aufnahmebehältnis typischerweise einen Befüllanschluss zum Befüllen mit Dialyselösung auf.

Bei dem Aufnahmebehältnis kann es sich um einen flexiblen Beutel oder um einen starren Behälter handeln. Im Falle eines starren Behälters kann ein Druckausgleichsventil vorgesehen sein. Es kann sich auch um einen flexiblen und vorzugsweise elastischen Beutel handeln, der in einem starren Behälter gelagert wird. Bei der Befüllung des Beutels füllt ein solcher Beutel idealerweise den Raum des umgebenden starren Behälters weitestgehend aus. Eine derartige Bag-In-Box-Verpackung hat den Vorteil, dass der typischerweise als Disposable ausgeführte Innenbeutel durch die Umverpackung vor mechanischen Einflüssen geschützt wird. Die mechanische Robustheit des Lösungsbeutels, hier Innenbeutels verliert gegenüber vorbekannten Lösungen an Bedeutung, wodurch eine materialsparende und kostengünstigere Ausführung ermöglicht wird. Insbesondere aufgrund der Tatsache, dass es sich bei Lösungsbeuteln typischerweise um Einwegprodukte handelt, ist die Kostenersparnis und die Abfallersparnis erheblich.

Der Behälter kann transparent sein oder ein transparentes Sichtfenster aufweisen. Gegebenenfalls können Füllstandsmarkierungen vorgesehen sein. Die Vorrichtung kann gegebenenfalls ein Dosierbehältnis aufweisen, das zwischen den Entnahmeanschlüssen und dem Aufnahmebehältnis angeordnet ist, um die Dosierung der richtigen Flüssigkeitsmenge für eine Behandlung zu vereinfachen.

In einer Ausführungsform ist vorgesehen,dass jedem Entnahmeanschluss ein Ventil und ein Drainageanschluss zugeordnet ist. So kann jeder Entnahmeanschluss auch für einen Draining-Vorgang genützt werden. Beispiele geeigneter Ventile umfassen Drehventile oder 3/3-Wege-Ventile. Nach Anschließen der in den Bauchraum des Patienten führenden Leitung an den Entnahmeanschluss kann das zuvor geschlossene Ventil zunächst so eingestellt werden oder das Ventil so eingestellt bleiben, dass verbrauchte Dialyselösung durch den Drainageanschluss bzw. eine daran angeschlossene Drainage-Leitung abfließt. Sodann kann das Ventil so eingestellt werden, dass frische Dialyselösung aus dem Aufnahmebehältnis in den Baurchraum des Patienten fließen kann. Gegebenenfalls kann zum Entlüften der Leitung auch eine Ventilstellung angenommen werden, in der ein Fluss von Dialyselösung aus dem Aufnahmebehältnis in den Drainageanschluss bzw. eine daran angeschlossene Drainage-Leitung ermöglicht wird.

In einer Ausführungsform ist vorgesehen,dass die Vorrichtung eine Entnahmehauptleitung aufweist, die mit dem Aufnahmebehältnis in Verbindung steht und von der mehrere Entnahmeeinzelleitungen mit den Entnahmeanschlüssen abgehen. Alternativ oder zusätzlich kann vorgesehen sein, dass in den Entnahmeeinzelleitungen jeweils Schließmittel angeordnet sind, mit denen die Entnahmeeinzelleitungen verschlossen werden können. Bei den Schließmitteln kann es sich beispielsweise um Klemmen handeln, mit denen die jeweilige Leitung abgeklemmt werden kann. In einer Ausführungsform ist vorgesehen, dass die Schließmittel und vorzugsweise Klemmen so ausgebildet sind, dass die jeweilige Leitung irreversibel verschlossen werden kann. In einer Ausführungsform ist vorgesehen, dass die Schließmittel so ausgebildet sind, dass sie sich bei oder nach einer Abkopplung der Entnahmeeinzelleitung vom Patienten automatisch verschließen. In einer Ausführungsform sind die Schließmittel derart ausgebildet, dass eine erneute Öffnung der benutzten Entnahmeeinzelleitung unmöglich ist.

In einer Ausführungsform ist vorgesehen,dass die Entnahmeeinzelleitungen an unterschiedlichen Verzweigungspunkten von der Entnahmehauptleitung abzweigen, die über die Länge der Entnahmehauptleitung verteilt sind. Das Leitungssystem bestehend aus der Entnahmehauptleitung und den Entnahmeeinzelleitungen hat in dieser Ausführungsform eine Baumstruktur, wobei die Entnahmehauptleitung dem Stamm und die Entnahmeeinzelleitungen den Ästen entsprechen. Jedem Entnahmeanschluss kann eine Einzelleitung zugeordnet sein.

In einer Ausführungsform ist vorgesehen,dass in der Entnahmehauptleitung jeweils Schließmittel zwischen aufeinanderfolgenden Abzweigungspunkten der Entnahmeeinzelleitung angeordnet sind, mit denen die Entnahmehauptleitung verschlossen werden können.

Die Schließmittel können wir oben näher beschrieben ausgebildet sein.

In der Anwendung kann vorgesehen sein, bei der ersten Flüssigkeitsentnahme denjenigen Entnahmeanschluss zu verwenden, der an der letzten, d.h., der am weitesten von dem Aufnahmebehältnis entfernten Entnahmeeinzelleitung sitzt. Nach Beendigung dieses Vorgangs kann die betreffende Entnahmeeinzelleitung unter Verwendung eines Schließmittels, z.B. einer Klemme, eines Ventils oder eines Hahns verschlossen werden. Alternativ kann die Entnahmehauptleitung an einer Stelle zwischen den Abzeigungen der letzten und vorletzten Entnahmeeinzelleitungen unter Verwendung eines Schließmittels verschlossen werden. Bei der zweiten Flüssigkeitsentnahme kann sodann der vorletzte Entnahmeanschluss verwendet werden und im Anschluss auch dieser durch Verschließen der Entnahmeeinzelleitung oder Entnahmehauptleitung abgekoppelt werden. Dieser Vorgang kann wiederholt werden, bis der letzte Entnahmeanschluss an der letzten, d.h., am nähesten am Aufnahmebehältnis befindlichen Entnahmeeinzelleitung benutzt oder die gesamte Dialyselösung im Aufnahmebehältnis verbraucht wurde. Eine Abkopplung an der Entnahmehauptleitung kann den Vorteil haben, dass weniger Totvolumen entsteht.

In einer Ausführungsform ist vorgesehen,dass die Entnahmehauptleitung in einen Verteiler mündet und die Entnahmeeinzelleitungen sternförmig von dem Verteiler abgehen. Der Verteiler kann einen Schalter aufweisen, der so ausgebildet ist, dass immer nur die Freigabe einer einzelnen Entnahmeeinzelleitung möglich ist und dass nur eine solche Entnahmeeinzelleitung freigegeben werden kann, welche zuvor noch nicht verwendet wurde. Ein Beispiel eines geeigneten Schalters umfasst einen Drehschalter, der nur in eine Richtung gedreht werden kann.

Am vorlaufseitigen Ende der Entnahmehauptleitung kann ein Verbindungsmechanismus vorgesehen sein, der ausgebildet ist, um das Leitungssystem insgesamt von dem Aufnahmebehältnis abkoppeln zu können. So kann beispielsweise entweder das Aufnahmebehältnis oder das Leitungssystem wiederverwendet werden.

Gegebenenfalls kann die Entnahmehauptleitung und/oder können die Entnahmeeinzelleitungen Rückschlagventile als zusätzliche Kontaminationsbarriere aufweisen.

In einer Ausführungsform ist vorgesehen,dass die Vorrichtung eine Entnahmeleitung aufweist, an der die Entnahmeanschlüsse in Serie angeordnet sind, wobei vorzugsweise vorgesehen ist, dass die Entnahmeleitung in mehrere abkoppelbare Sektoren unterteilt ist und in jedem der Sektoren ein Entnahmeanschluss angeordnet ist. In den einzelnen Sektoren können wie oben beschriebene Schließmittel angeordnet sein, um die Sektoren abkoppeln zu können. Die Sektoren können anhand von Verbindern miteinander verbunden sein, die Solltrennstellen aufweisen, um einen Sektor mit einem verbrauchten Entnahmeanschluss abzutrennen. Vorzugsweise sind die Solltrennstellen so ausgebildet, dass der Durchlass beim Trennen verschlossen wird.

In einer Ausführungsform ist vorgesehen,dass die Entnahmeanschlüsse mit luftdichten Umhüllungen einzeln versiegelt sind, wobei vorzugsweise vorgesehen ist, dass eine der Anzahl der Entnahmeanschlüssen entsprechende Anzahl an Umhüllungen vorhanden ist, wobei eine innerste Umhüllung einen Entnahmeanschluss und jede weitere Umhüllung die bestehende Umhüllung und einen weiteren Entnahmeanschluss umschließt. Geeignete Umhüllungen umfassen beispielsweise Kunststoffbeutel, Kappen oder dergleichen. Es kann vorgesehen sein, dass die Umhüllungen dem Prinzip einer russischen Puppe folgen. Wird die äußerste Umhüllung geöffnet, wird ein Entnahmeanschluss sowie die nächste Umhüllung freigegeben, die alle weiteren Entnahmeanschlüsse versiegelt. Wird diese nächste Umhüllung geöffnet, wird ein weiterer Entnahmeanschluss und die übernächste Umhüllung freigegeben, und so weiter. Erst nach Öffnen der innersten Umhüllung wird der letzte Entnahmeanschluss freigegeben. Durch diese Art der Versiegelung kann die Gefahr der Verwechslung von bereits benutzten und frischen Anschlüssen minimiert werden. Im Falle des oben vorgestellten Leitungssystems mit Entnahmehauptleitung und Entnahmeeinzelleitungen in Baumstruktur kann der letzte Entnahmeanschluss an der letzten, d.h., am nähesten am Aufnahmebehältnis befindlichen Entnahmeeinzelleitung von allen und der erste Entnahmeanschluss an der ersten, d.h., am weitesten vom Aufnahmebehältnis entfernten Entnahmeeinzelleitung von nur der äußersten Umhüllung umgeben sein.

Bei der Doppelverwendungssicherung kann beispielsweise eine bewegliche Hülse umfassen, die von einer offenen Stellung in eine geschlossene Stellung bewegt werden kann und in geschlossener Stellung den Entnahmeanschluss blockiert. Die Hülse kann beispielsweise so ausgebildet sein, dass sie in axialer Richtung des Anschlusses von einer offenen Stellung in eine geschlossene Stellung verschoben werden kann. Es kann vorgesehen sein, dass die geschlossene Stellung irreversibel ist, d.h., dass die Hülse nicht mehr von der geschlossenen in die offene Stellung zurückverschoben werden kann. Dieser Arrest kann beispielsweise durch einen Rastvorsprung und eine Rastnase realisisert sein. So kann eine unerwünschte Mehrfachverwendung desselben Anschlusses effektiv vermieden werden. Die Hülse kann beispielsweise gegen die geschlossene Stellung vorgespannt sein, sodass sie nach Aufbrechen eines Halteelements automatisch in die geschlossene Stellung übergeführt wird. Das Halteelement kann so ausgebildet sein, dass es bei Ankopplung der in den Bauchraum des Patienten führenden Leitung aufgebrochen wird. Die Hülse wird dann so lange von der Patientenleitung gehalten, wie diese angeschlossen ist. Bei Abnahme schließt die Hülse irreversibel.

Das Behältnis kann keine gebrauchsfertige Dialyselösung sondern lediglich ein Konzentrat enthalten. Die gebrauchsfertige Dialyselösung kann vor Ort durch Auffüllen des Behältnisses mit aufgereinigtem sterilen Wasser, beispielsweise aus einer Revers-Osmose-Anlage, hergestellt werden. In einer solchen Ausführungsform besitzt das Behältnis auch eine Zuleitung für Flüssigkeiten, wobei die Zuleitung vorzugsweise verschließbar ist. Das Konzentrat kann in dem Behältnis frei oder in einem separaten Kompartiment vorgelegt werden. Es können auch mehrere verschiedene Konzentrate in mehreren Kompartimenten vorgelegt werden. Durch eine Brechverbindung kann Aufbrechen eine fluide Verbindung zwischen den Konzentratkompartimenten und einem Mischkompartiment hergestellt werden. Als Brechverbindung eignen sich für den Fall, dass das Behältnis ein Folienbeutel ist beispielsweise peelfähige Schweißnähte.

Weitere Einzelheiten und Vorteile der Erfindung ergeben sich aus den nachfolgend anhand der Figuren beschriebenen Ausführungsbeispielen. In den Figuren zeigen:
- Figur 1:: eine erfindungsgemäße Vorrichtung mit einem Leitungssystem bestehend aus einer Entnahmehauptleitung und mehreren Entnahmeeinzelleitungen in Baumstruktur;
- Figur 2:: eine Variante eines Entnahmeanschlusses mit Drainage-Anschluss und 3-Wege-Ventil;
- Figur 3:: eine Variante eines Entnahmeanschlusses mit Drainage-Anschluss und Drehschalter;
- Figur 4:: die Vorrichtung der Figur 1 mit einer Matryoschka-Umhüllung der Entnahmeanschlüsse;
- Figur 5:: eine erfindungsgemäße Vorrichtung mit einem Leitungssystem bestehend aus einer Entnahmehauptleitung und mehreren Entnahmeeinzelleitungen in Sternstruktur;
- Figur 6:: eine erfindungsgemäße Vorrichtung mit einem Leitungssystem bestehend aus einer Entnahmeleitung mit mehreren in Serie angeordneten Entnahmesektoren; und
- Figur 7:: eine Variante eines Entnahmeanschlusses mit Doppelverwendungssicherung.

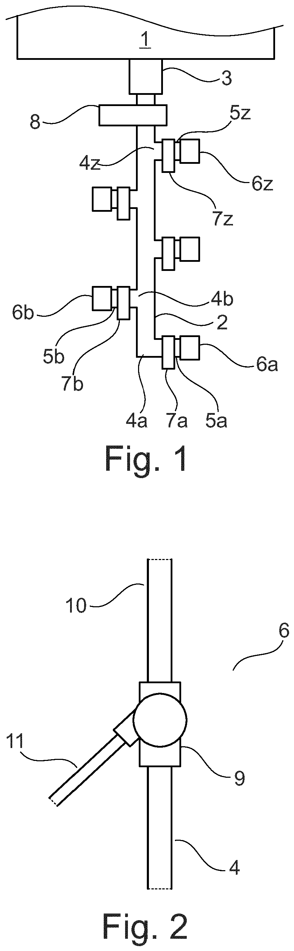

Eine erste Variante einer erfindungsgsmäßen Vorrichtung ist in Figur 1 gezeigt. Sie umfasst ein Aufnahmebehältnis in Form eines Folienbeutels 1, dessen Dimensionen so sind, das mehrere Portionen von jeweils etwa zwei Litern an Peritonealdialyselösung aufgenommen werden können. Der Folienbeutel 1 ist mit einer Entnahmehauptleitung 2 anhand einer Verbindung 3 mit korrespondierenden lösbaren Verbindungspartnern realisiert ist, wodurch die Entnahmehauptleitung 2 vom Folienbeutel 1 abgenommen werden kann.

Von der Entnahmehauptleitung 2 zweigt an unterschiedlichen Verweigungspunkten 4a, 4b, etc., die in Serie über die Länge der Entnahmehauptleitung verteilt sind, jeweils eine Entnahmeeinzelleitung 5a, 5b, etc. ab. Jede der Entnahmeeinzelleitungen weist an dessen Ende einen Entnahmeanschluss 6a, 6b, etc. auf und ist ferner mit einer Klemme 7a, 7b, etc. versehen.

In der Entnahmehauptleitung 2 ist zwischen dem am nähesten am Beutel befindlichen Verzweigungspunkt 4z und dem Verbindungselementepaar 3 eine Hauptklemme 8 vorgesehen.

Die einzelnen Entnahmeanschlüsse 6 können so ausgebildet sein wie in Figur 2 dargestellt und ein Y-Ventil 9 umfassen, an dem die Entnahmeeinzelleitung 4 fest verbunden ist und das Konnektoren zum Anschluss einer Patientenleitung 10 und einer Drainage-Leitung 11 aufweist. So kann der Entnahmeanschluss 6 auch für einen Draining-Vorgang genützt werden, wobei nach dem Anschließen der Patientenleitung 10 und der Drainage-Leitung 11 das Ventil 9 zunächst so eingestellt wird, dass verbrauchte Dialyselösung aus der Patientenleitung 10 in die Drainage-Leitung 11 abfließt. Sodann das Ventil 9 so eingestellt werden, dass frische Dialyselösung aus der Entnahmeeinzelleitung 4 in die Patientenleitung 10 fließen kann.

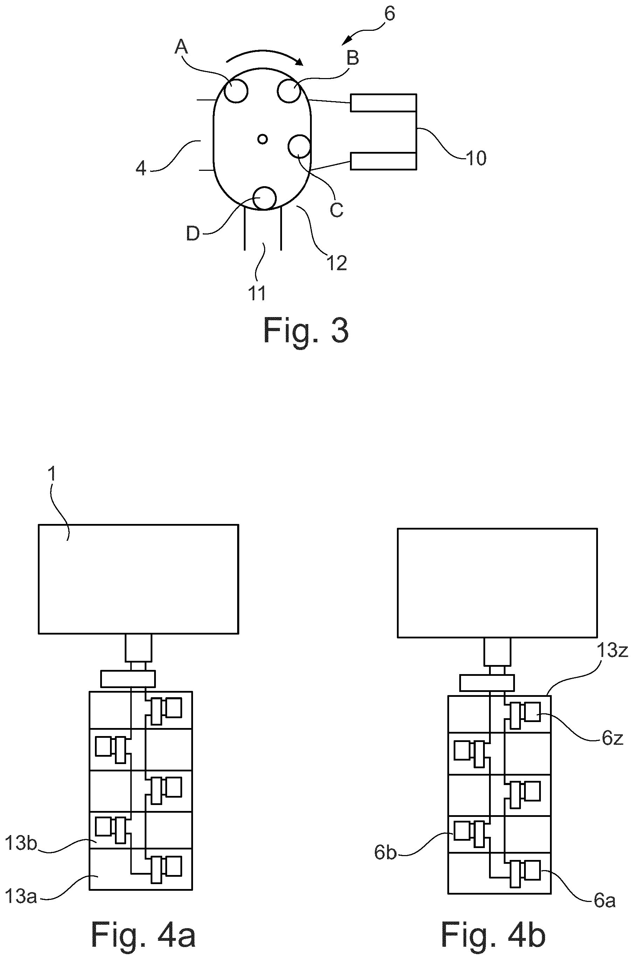

Die einzelnen Entnahmeanschlüsse 6 können auch so ausgebildet sein wie in Figur 3 dargestellt und einen Drehschalter 12 umfassen, der vier Positionen durchlaufen kann. In Position A ist der Drehschalter 12 geschlossen. In Position B wird ein Abfluss verbrauchter Dialyselösung aus der Patientenleitung 10 in die Drainage-Leitung 11 ermöglicht. In Position C kann frische Dialyselösung aus der Entnahmeeinzelleitung 4 in die Patientenleitung 10 fließen. In Position D ist der Drehschalter wieder geschlossen. Eine Drehung des Schalters 12 ist lediglich im Uhrzeigersinn möglich, wodurch eine hohe Bediensicherheit erreicht wird.

In der Anwendung der in Figur 1 gezeigten Vorrichtung kann vorgesehen sein, bei der ersten Flüssigkeitsentnahme den Entnahmeanschluss 6a zu verwenden, der an der letzten, d.h., der am weitesten von dem Aufnahmebehältnis entfernten Entnahmeeinzelleitung 5a sitzt. Nach Beendigung dieses Vorgangs kann die betreffende Entnahmeeinzelleitung 5a unter Verwendung der Klemme 7a verschlossen werden. Bei der zweiten Flüssigkeitsentnahme kann sodann der vorletzte Entnahmeanschluss 6b verwendet werden und im Anschluss auch dieser durch Verschließen der Entnahmeeinzelleitung 5b anhand der Klemme 7b abgekoppelt werden. Dieser Vorgang kann wiederholt werden, bis der letzte Entnahmeanschluss 6z an der letzten, d.h., am nähesten am Aufnahmebehältnis befindlichen Entnahmeeinzelleitung 5z benutzt oder die gesamte Dialyselösung im Aufnahmebeutel verbraucht wurde.

Figur 4 zeigt eine Möglichkeit, wie die Entnahmeanschlüsse 6a, 6b, etc. der Vorrichtung gemäß Figur 1 in einer Weise versiegelt werden können, dass die Gefahr der Verwechslung von bereits benutzten und frischen Anschlüssen minimiert werden kann. Nämlich ist eine der Anzahl der Entnahmeanschlüsse 6a, 6b, etc. entsprechende Anzahl an Folienumhüllungen 13a, 13b, etc. vorgesehen, die einander wie russische Puppen umgeben. Die innerste Umhüllung 13z umgibt ausschließlich den innersten Entnahmeanschluss 6z und jede weitere Umhüllung die bestehende Umhüllung und einen weiteren Entnahmeanschluss. Wird die äußerste Umhüllung 13a, ausgehend von der vollständig geschlossenen Stellung der Figur 4a, von einem Benutzer geöffnet, wird der erste Entnahmeanschluss 6a sowie die nächste Umhüllung 13b freigegeben, die alle weiteren Entnahmeanschlüsse 6b etc. versiegelt. Wird diese nächste Umhüllung 13b geöffnet, wird ein weiterer Entnahmeanschluss und die übernächste Umhüllung freigegeben, wie in Figur 4b dargestellt, und so weiter. Erst nach Öffnen der innersten Umhüllung 13z wird der letzte Entnahmeanschluss 6z freigegeben.

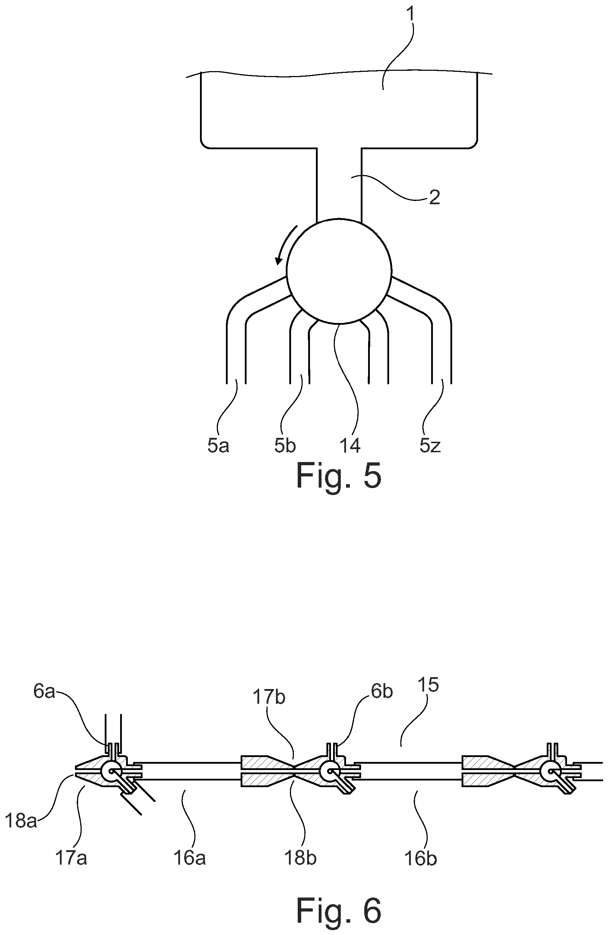

Figur 5 zeigt eine Variante der Erfindung, in welcher die Entnahmehauptleitung 2 in einen Verteiler 14 mündet und die Entnahmeeinzelleitungen 5a, 5b, etc. sternförmig von dem Verteiler 14 abgehen. Der Verteiler 14 weist einen Drehschalter auf, der so ausgebildet ist, dass er nur gegen den Uhrzeigersinn gedreht werden kann, wie dies in der Figur mit einem Pfeil angedeutet ist. In der Trommel des Drehschalters ist eine Leitung angeordnet, sodass immer nur die Freigabe einer einzelnen Entnahmeeinzelleitung 5a, 5b, etc. möglich ist. Durch die Unmöglichkeit eines Zurückdrehens im Uhrzeigersinn kann zudem immer nur eine solche Entnahmeeinzelleitung 5a, 5b, etc. freigegeben werden kann, zuvor noch nicht verwendet wurde. Obwohl in der Figur nicht näher dargestellt, sind die Entnahmeeinzelleitungen mit Entnahmeanschlüssen und Klemmen ausgestattet, wie dies im Zusammenhang mit Figur 1 näher erläutert wurde.

Figur 6 zeigt eine Variante der Erfindung mit einem Leitungssystem bestehend aus einer Entnahmeleitung 15 mit mehreren in Serie angeordneten Entnahmesektoren 16a, 16b, etc., die anhand von Verbindern 17a, 17b, etc. miteinander verbunden sind. Die Entnahmeleitung 15 ist an ihrem in der Figur 6 rechten Ende mit dem Lösungsbeutel verbunden, wie dies im Zusammenhang mit Figur 1 für die Entnahmehauptsleitung erklärt wurde. Die Verbinder 17a, 17b, etc. umfassen jeweils Entnahmeanschlüsse 6a, 6b, etc., die wie im Zusammenhang mit Figur 2 erklärt ausgebildet sind. Ferner umfassen die Verbinder 17a, 17b, etc. je eine Solltrennstelle 18a, 18b, etc., um die Entnahmesektoren 16a, 16b, etc. mit verbrauchten Entnahmeanschlüsse 6a, 6b, etc. abkoppeln zu können. Beispielsweise kann der Verbinder 17b an der Solltrennstelle 18b gekappt werden, nachdem der Entnahmeanschluss 6a für eine Behandlung in Gebrauch war und dieser Gebrauch beendet wurde. Das Kappen der Verbindung an den Solltrennstellen 18a, 18b, etc. kann grundsätzlich in beliebiger Weise wie beispielsweise durch Brechen erfolgen, wobei die Solltrennstellen 18a, 18b, etc. vorzugsweise so ausgebildet sind, dass beim Abkoppeln die zum abgekoppelten Sektor führende Leitung verschlossen wird, um ein Ausfließen von frischer Dialsyslösung zu verhindern.

Auch in den Varianten der Figuren 5 und 6 kann eine Versiegelung der Entnahmeanschlüsse 6a, 6b, etc. so erfolgen, wie dies im Zusammenhang mit Figur 4 für die Variante der Figur 1 erklärt wurde.

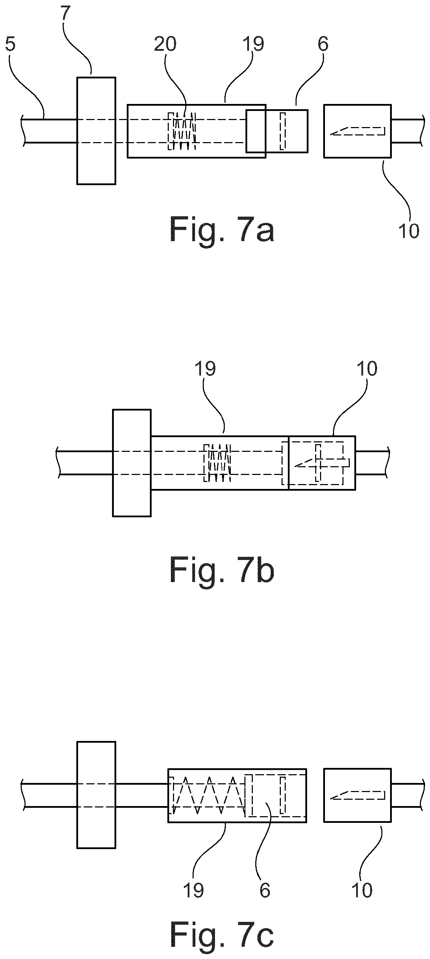

Letztlich zeigt Figur 7 eine mögliche Ausgestaltung der Entnahmeanschlüsse 6 mit einer Hülse19 zur Vermeidung einer Verwendung eines bereits verwendeten Anschlusses. Die Hülse 19 ist so ausgebildet, dass sie in axialer Richtung des Anschlusses 6 von einer in Figur 7a gezeigten offenen Stellung in eine in Figur 7c gezeigte geschlossene Stellung geschoben werden kann, wobei in der geschlossenen Stellung der Entnahmeanschluss 6 blockiert wird. Die geschlossene Stellung ist irreversibel, da die Hülse 19 eine in der Figur nicht näher dargestellte Rastnase aufweist, die in der geschlossenen Stellung hinter bzw. in der Figur rechts von einem in der Figur ebenfalls nicht näher dargestellten Rastvorsprung zu liegen kommt. Die Hülse 19 ist mit einer Feder 20 gegen die geschlossene Stellung vorgespannt. Wird eine Patientenleitung 10 auf den Anschluss 6 gesteckt, so wird die Hülse nach hinten bzw. in der Figur nach links in eine Stellung gedrückt, die in Figur 7b gezeigt ist. Dabei wird eine in der Figur nicht näher dargestellte Haltebrücke aufgebrochen, welche die Hülse 19 zuvor, d.h. im Zustand der Figur 7a gegen die Vorspannung in ihrer offenen Stellung hält. Solange die Patientenleitung 10 am Anschluss 6 verbleibt, wie in Figur 7b gezeigt, wird die Hülse 19 in dieser Positon gehalten. Bei Abnahme der Patientenleitung 10 vom Anschluss 6 wird die Hülse 19 dann von der Feder 20 nach rechts in die in Figur 7c gezeigte Stellung gedrückt und schließt irreversibel.

## Patentansprüche

1. Vorrichtung enthaltend eine Dialyselösung oder ein Dialyselösungskonzentrat, vorzugsweise zur Verwendung in der Peritonealdialyse, mit einem Aufnahmebehältnis, das ein Lösungsvolumen fassen kann, welches für wenigstens zwei Behandlungen bestimmt ist,
**dadurch gekennzeichnet,**
**dass** die Vorrichtung mehrere Entnahmeanschlüsse zur Entnahme der Dialyselösung aus dem Aufnahmebehältnis aufweist und die Entnahmeanschlüsse eine Doppelverwendungssicherung aufweisen, die den Entnahmeanschluss nach Verwendung irreversibel verschließt..

2. Vorrichtung nach Anspruch 1, **dadurch gekennzeichnet, dass** jedem Entnahmeanschluss ein Ventil und ein Drainageanschluss zugeordnet ist.

3. Vorrichtung nach einem der vorhergehenden Ansprüche, **dadurch gekennzeichnet, dass** die Vorrichtung eine Entnahmehauptleitung aufweist, die mit dem Aufnahmebehältnis in Verbindung steht und von der mehrere Entnahmeeinzelleitungen mit den Entnahmeanschlüssen abgehen.

4. Vorrichtung nach Anspruch 3, **dadurch gekennzeichnet, dass** in den Entnahmeeinzelleitungen jeweils Schließmittel angeordnet sind, mit denen die Entnahmeeinzelleitungen verschlossen werden können.

5. Vorrichtung nach Anspruch 4, **dadurch gekennzeichnet, dass** die Schließmittel so ausgebildet sind, dass sie sich bei oder nach einer Abkopplung der Entnahmeeinzelleitung vom Patienten automatisch verschließen.

6. Vorrichtung nach einem der Ansprüche 3 bis 5, **dadurch gekennzeichnet, dass** die Entnahmeeinzelleitungen an unterschiedlichen Verweigungspunkten von der Entnahmehauptleitung abzweigen, die über die Länge der Entnahmehauptleitung verteilt sind.

7. Vorrichtung nach Anspruch 6, **dadurch gekennzeichnet, dass** in der Entnahmehauptleitung jeweils Schließmittel zwischen aufeinanderfolgenden Abzweigungspunkten der Entnahmeeinzelleitung angeordnet sind, mit denen die Entnahmehauptleitung verschlossen werden können.

8. Vorrichtung nach einem der Ansprüche 3 bis 5, **dadurch gekennzeichnet, dass** die Entnahmehauptleitung in einen Verteiler mündet und die Entnahmeeinzelleitungen sternförmig von dem Verteiler abgehen.

9. Vorrichtung nach Anspruch 1 oder 2, **dadurch gekennzeichnet, dass** die Vorrichtung eine Entnahmeleitung aufweist, an der die Entnahmeanschlüsse in Serie angeordnet sind, wobei vorzugsweise vorgesehen ist, dass die Entnahmeleitung in mehrere abkoppelbare Sektoren unterteilt ist und in jedem der Sektoren ein Entnahmeanschluss angeordnet ist.

10. Vorrichtung nach einem der vorhergehenden Ansprüche, **dadurch gekennzeichnet, dass** die Entnahmeanschlüsse mit luftdichten Umhüllungen einzeln versiegelt sind, wobei vorzugsweise vorgesehen ist, dass eine der Anzahl der Entnahmeanschlüssen entsprechende Anzahl an Umhüllungen vorhanden ist, wobei eine innerste Umhüllung einen Entnahmeanschluss und jede weitere Umhüllung die bestehende Umhüllung und einen weiteren Entnahmeanschluss umschließt.

## Claims

1. An apparatus containing a dialysis solution or a dialysis solution concentrate, preferably for use in peritoneal dialysis, comprising a reception container that can accommodate a solution volume which is intended for at least two treatments,
**characterized in that**
the apparatus has a plurality of extraction ports for extracting the dialysis solution from the reception container; and **in that** the extraction ports have a security against double use that irreversibly closes the extraction port after use.

2. An apparatus in accordance with claim 1, **characterized in that** a valve and a drainage port are associated with each extraction port.

3. An apparatus in accordance with one of the preceding claims, **characterized in that** the apparatus has a main extraction line that is connected to the reception container and from which a plurality of single extraction lines having the extraction ports lead off.

4. An apparatus in accordance with claim 3, **characterized in that** respective closing means by which the single extraction lines can be closed are arranged in the single extraction lines.

5. An apparatus in accordance with claim 4, **characterized in that** the closing means are configured such that they close automatically on or after a decoupling of the single extraction line from the patient.

6. An apparatus in accordance with one of the claims 3 to 5, **characterized in that** the single extraction lines branch off from the main extraction line at different branch points that are distributed over the length of the main extraction line.

7. An apparatus in accordance with claim 6, **characterized in that** respective closing means are arranged in the main extraction line between branch points of the single extraction line that follow one another and with which the main extraction line can be closed.

8. An apparatus in accordance with one of the claims 3 to 5, **characterized in that** the main extraction line opens into a distributor and the single extraction lines lead off from the distributor in star shape.

9. An apparatus in accordance with claim 1 or claim 2, **characterized in that** the apparatus has an extraction line at which the extraction ports are arranged in series, with provision preferably being made that the extraction line is divided into a plurality of decouplable sectors and an extraction port is arranged in each of the sectors.

10. An apparatus in accordance with one of the preceding claims, **characterized in that** the extraction ports are individually sealed by airtight sheaths, with provision preferably being made that a number of sheaths corresponding to the number of extraction ports is present, with an innermost sheath surrounding an extraction port and every further sheath surrounding the existing sheath and a further extraction port.

## Revendications

1. Dispositif contenant un dialysat ou un concentré pour dialysat, de préférence destiné à être utilisé dans la dialyse péritonéale, comprenant un contenant de réception, qui peut renfermer un volume de solution qui est prévu pour au moins deux traitements,
**caractérisé en ce que**
le dispositif comporte plusieurs raccords de prélèvement pour le prélèvement du dialysat dans le contenant de réception et les raccords de prélèvement comportent une sécurité contre une double utilisation, qui ferme le raccord de prélèvement de manière irréversible après l'utilisation.

2. Dispositif selon la revendication 1, **caractérisé en ce qu'**une valve et un raccord de purge sont associés à chaque raccord de prélèvement.

3. Dispositif selon l'une des revendications précédentes, **caractérisé en ce que** le dispositif comporte une conduite principale de prélèvement qui est reliée au contenant de réception et de laquelle partent plusieurs conduites individuelles de prélèvement avec les raccords de prélèvement.

4. Dispositif selon la revendication 3, **caractérisé en ce que** des moyens de fermeture sont disposés dans chaque conduite individuelle de prélèvement, avec lesquels les conduites individuelles de prélèvement peuvent être fermées.

5. Dispositif selon la revendication 4, **caractérisé en ce que** les moyens de fermeture sont conçus de manière à se fermer automatiquement lors de ou après un désaccouplement de la conduite individuelle de prélèvement du patient.

6. Dispositif selon l'une des revendications 3 à 5, **caractérisé en ce que** les conduites individuelles de prélèvement bifurquent en différents points de branchement de la conduite principale de prélèvement, lesdits points de branchement étant répartis sur la longueur de la conduite principale de prélèvement.

7. Dispositif selon la revendication 6, **caractérisé en ce que** des moyens de fermeture sont disposés respectivement dans la conduite principale de prélèvement entre des points de branchement se suivant de la conduite individuelle de prélèvement, lesdits moyens de fermeture permettant de fermer la conduite principale de prélèvement.

8. Dispositif selon l'une des revendications 3 à 5, **caractérisé en ce que** la conduite principale de prélèvement débouche dans un élément de répartition et les conduites individuelles de prélèvement partent en étoile de l'élément de répartition.

9. Dispositif selon la revendication 1 ou 2, **caractérisé en ce que** le dispositif comporte une conduite de prélèvement, sur laquelle les raccords de prélèvement sont disposés en série, dans lequel il est de préférence prévu que la conduite de prélèvement soit divisée en plusieurs secteurs pouvant être désaccouplés et qu'un raccord de prélèvement soit disposé dans chacun des secteurs.

10. Dispositif selon l'une des revendications précédentes, **caractérisé en ce que** les raccords de prélèvement sont scellés individuellement avec des enveloppes étanches à l'air, dans lequel il est de préférence prévu qu'il y ait un nombre d'enveloppes correspondant au nombre de raccords de prélèvement, une enveloppe la plus à l'intérieur renfermant un raccord de prélèvement et chaque enveloppe supplémentaire renfermant l'enveloppe existante et un autre raccord de prélèvement.
